(19) Europäisches Patentamt
European Patent Office
Office européen des brevets

(11) **EP 2 138 138 B1**

(12) **EUROPÄISCHE PATENTSCHRIFT**

(45) Veröffentlichungstag und Bekanntmachung des Hinweises auf die Patenterteilung:
**25.10.2017 Patentblatt 2017/43**

(51) Int Cl.:
*A61F 9/008* *(2006.01)*  *A61F 9/009* *(2006.01)*

(21) Anmeldenummer: **09172932.7**

(22) Anmeldetag: **23.07.2004**

(54) **Vorrichtung zum Ausbilden gekrümmter Schnittflächen in einem transparenten Material**

Device for forming curved cuts in a transparent material

Dispositif pour former des surfaces de coupe courbes dans une matière transparente

(84) Benannte Vertragsstaaten:
**AT BE BG CH CY CZ DE DK EE ES FI FR GB GR HU IE IT LI LU MC NL PL PT RO SE SI SK TR**

(30) Priorität: **25.07.2003 DE 10334110**

(43) Veröffentlichungstag der Anmeldung:
**30.12.2009 Patentblatt 2009/53**

(60) Teilanmeldung:
**10182173.4 / 2 260 805**

(62) Dokumentnummer(n) der früheren Anmeldung(en) nach Art. 76 EPÜ:
**04763451.4 / 1 648 360**

(73) Patentinhaber: **Carl Zeiss Meditec AG**
**07745 Jena (DE)**

(72) Erfinder:
• **Mühlhoff, Dirk**
**07751 Jena (DE)**
• **Gerlach, Mario**
**16548 Glienicke-Nordbahn (DE)**
• **Sticker, Markus**
**07749 Jena (DE)**
• **Lang, Carsten**
**07607 Eisenberg (DE)**
• **Bischoff, Mark**
**07749 Jena (DE)**
• **Bergt, Michael**
**99425 Weimar (DE)**

(74) Vertreter: **Patentanwälte Geyer, Fehners & Partner mbB**
**Perhamerstrasse 31**
**80687 München (DE)**

(56) Entgegenhaltungen:
WO-A-94/09849    US-A- 5 993 438
US-A- 6 110 166    US-B1- 6 325 792

EP 2 138 138 B1

## Beschreibung

Beschrieben wird ein Verfahren zum Ausbilden gekrümmter Schnittflächen in einem transparenten Material, insbesondere in der Augenhornhaut, durch Erzeugen optischer Durchbrüche im Material mittels ins Material fokussierter Laserstrahlung, wobei der Fokuspunkt dreidimensional verstellt wird, um die Schnittfläche durch Aneinanderreihung der optischen Durchbrüche zu bilden, und wobei die Verstellung des Fokuspunktes in einer ersten Raumrichtung mit geringer Maximalgeschwindigkeit erfolgt, als in den übrigen zwei Raumrichtungen. Die Erfindung bezieht sich auf eine Vorrichtung zum Ausbilden gekrümmter Schnittflächen in einem transparenten Material, insbesondere in der Augenhornhaut, mit einer Laserstrahlungsquelle, die Laserstrahlung in das Material fokussiert und dort optische Durchbrüche bewirkt, wobei eine Scaneinrichtung, die den Fokuspunkt dreidimensional verstellt, und eine Steuereinrichtung vorgesehen sind, die die Scaneinrichtung ansteuert, um die Schnittfläche durch Aneinanderreihen der optischen Durchbrüche im Material zu bilden, und wobei die Scaneinrichtung zur Verstellung des Fokuspunktes in einer Raumrichtung eine verstellbare Optik aufweist.

Gekrümmte Schnittflächen innerhalb eines transparenten Materials werden insbesondere bei laserchirurgischen Verfahren und dort insbesondere bei augenchirurgischen Eingriffen erzeugt. Dabei wird Behandlungs-Laserstrahlung innerhalb des Gewebes d.h. unterhalb der Gewebeoberfläche derart fokussiert, daß optische Durchbrüche im Gewebe entstehen.

Im Gewebe laufen dabei zeitlich hintereinander mehrere Prozesse ab, die durch die Laserstrahlung initiiert werden. Überschreitet die Leistungsdichte der Strahlung einen Schwellwert, kommt es zu einem optischen Durchbruch, der im Material eine Plasmablase erzeugt. Diese Plasmablase wächst nach Entstehen des optischen Durchbruches durch sich ausdehnende Gase. Wird der optische Durchbruch nicht aufrechterhalten, so wird das in der Plasmablase erzeugte Gas vom umliegenden Material aufgenommen, und die Blase verschwindet wieder. Dieser Vorgang dauert allerdings sehr viel länger, als die Entstehung der Blase selbst. Wird ein Plasma an einer Materialgrenzfläche erzeugt, die durchaus auch innerhalb einer Materialstruktur liegen kann, so erfolgt ein Materialabtrag von der Grenzfläche. Man spricht dann von Photoablation. Bei einer Plasmablase, die vorher verbundene Materialschichten trennt, ist üblicherweise von Photodisruption die Rede. Der Einfachheit halber werden all solche Prozesse hier unter dem Begriff optischer Durchbruch zusammengefaßt, d.h. dieser Begriff schließt nicht nur den eigentlichen optischen Durchbruch sondern auch die daraus resultierenden Wirkungen im Material ein.

Für eine hohe Genauigkeit eines laserchirurgischen Verfahrens ist es unumgänglich, eine hohe Lokalisierung der Wirkung der Laserstrahlen zu gewährleisten und Kolateralschäden in benachbartem Gewebe möglichst zu vermeiden. Es ist deshalb im Stand der Technik üblich, die Laserstrahlung gepulst anzuwenden, so daß der zur Auslösung eines optischen Durchbruchs nötige Schwellwert für die Leistungsdichte nur in den einzelnen Pulsen überschritten wird. Die US 5.984.916 zeigt diesbezüglich deutlich, daß der räumliche Bereich des optischen Durchbruchs (in diesem Fall der erzeugten Wechselwirkung) stark von der Pulsdauer abhängt. Eine hohe Fokussierung des Laserstrahls in Kombination mit sehr kurzen Pulsen erlaubt es damit, den optischen Durchbruch sehr punktgenau in einem Material einzusetzen.

Der Einsatz von gepulster Laserstrahlung hat sich in der letzten Zeit besonders zur laserchirurgischen Fehlsichtigkeitskorrektur in der Ophthalmologie durchgesetzt. Fehlsichtigkeiten des Auges rühren oftmals daher, daß die Brechungseigenschaften von Hornhaut und Linse keine optimale Fokussierung auf der Netzhaut bewirken.

Die erwähnte US 5.984.916 sowie die US 6.110.166 beschreiben gattungsgemäße Verfahren zur Schnitterzeugung mittels geeigneter Erzeugung optischer Durchbrüche, so daß im Endeffekt die Brechungseigenschaften der Hornhaut gezielt beeinflußt werden. Eine Vielzahl von optischen Durchbrüchen wird so aneinandergesetzt, daß innerhalb der Hornhaut des Auges ein linsenförmiges Teilvolumen isoliert wird. Das vom übrigen Hornhautgewebe getrennte linsenförmige Teilvolumen wird dann über einen seitlich öffnenden Schnitt aus der Hornhaut herausgenommen. Die Gestalt des Teilvolumens ist so gewählt, daß nach Entnahme die Form und damit die Brechungseigenschaften der Hornhaut so geändert sind, daß die erwünschte Fehlsichtigkeitskorrektur bewirkt ist. Die dabei geforderten Schnittflächen sind gekrümmt, was eine dreidimensionale Verstellung des Fokus nötig macht. Es wird deshalb eine zweidimensionale Ablenkung der Laserstrahlung mit gleichzeitiger Fokusverstellung in einer dritten Raumrichtung kombiniert.

Die zweidimensionale Ablenkung der Laserstrahlung ist wie die Fokusverstellung gleichermaßen für die Genauigkeit, mit der die Schnittfläche erzeugt werden kann, ausschlaggebend. Gleichzeitig wirkt sich die Verstellgeschwindigkeit, die dabei erreichbar ist, auf die Schnelligkeit, mit der die geforderte Schnittfläche erzeugt werden kann, aus. Eine schnelle Schnittflächenerzeugung ist nicht nur aus Komfort- oder Zeitersparniswünschen anzustreben, vor dem Hintergrund, daß bei ophthalmologischen Operationen unvermeidlicherweise Bewegungen des Auges auftreten, fördert eine schnelle Schnittflächenerzeugung zusätzlich die optische Qualität des erzielten Resultats bzw. senkt die Anforderungen an eventuelle Nachführungen von Augenbewegungen.

Die US 5993438 beschreibt eine Schnittflächenerzeugung mit Fokuspunktführung entlang einer Kreisspirale. Die WO 94/09849 sowie die US 6325792 offenbaren elliptische Ablation zur Fehlsichtigkeitskorrektur.

Der Erfindung liegt deshalb die Aufgabe zugrunde eine Vorrichtung der eingangs genannten Art so auszugestalten, daß für die Erzeugung einer Schnittfläche eine möglichst geringe Zeit erforderlich ist.

Diese Aufgabe wird erfindungsgemäß mit einer Vorrichtung nach Anspruch 1 gelöst.

Erfindungsgemäß werden also bei der Erzeugung der optischen Durchbrüche Bahnen verwendet, denen Höhenlinien der zu erzeugenden Schnittfläche zugrunde liegen. Die Höhenlinien sind dabei auf diejenige Raumrichtung des Systems bezogen, in der die langsamste Verstellgeschwindigkeit gegeben ist. Es ist dadurch möglich, den Fokus in dieser Raumrichtung über einen längeren Zeitraum nahezu unverändert zu lassen, und die höhere Verstellgeschwindigkeit in den anderen beiden Raumrichtungen kann ohne Begrenzung ausgeschöpft werden. Man erhält insgesamt eine schnelle Schnittflächenerzeugung. Die Höhenlinien kann man zweckmäßigerweise durch Schneiden der gekrümmten Schnittfläche mit Ebene senkrecht zur ersten Raumrichtung erhalten. Je exakter die Ebenen der Höhenlinien senkrecht zur ersten Raumrichtung stehen, desto konstanter kann die Verstellung in der ersten Raumrichtung während einer Höhenlinie gehalten werden.

Die Laserstrahlung wird dazu zumindest bezogen auf die zwei Raumrichtungen, die senkrecht zur Ebene der Höhenlinie liegen, unter Berücksichtigung des Höhenlinienverlaufes verstellt. Dabei ist es zum einen möglich, daß der Fokuspunkt innerhalb gewisser Toleranzen exakt der jeweiligen Höhenlinie folgt. In diesem Fall beschreibt der Fokuspunkt konzentrisch gelegene geschlossene Bahnkurven, wobei für jede Bahnkurve der Fokus in der ersten Raumrichtung entsprechend unterschiedlich eingestellt ist. Anstelle von geschlossenen Bahnkurven, die den Höhenlinien innerhalb bestimmter Toleranzen exakt folgen, ist es auch möglich, die Höhenlinien zusammenhängend miteinander zu verbinden. Der Fokuspunkt wird dabei entlang einer Höhenlinie bewegt, wobei die einzelnen Höhenlinien nicht als geschlossene Bahnkurven ausgeführt werden, sondern benachbarte Höhenlinien durch einen gleitenden Übergang miteinander verbunden sind, so daß insgesamt der Fokuspunkt auf einer einzigen zusammenhängenden Bahnkurve bewegt wird. Es entsteht dadurch eine auf einer geschlossenen Bahnkurve liegende Reihe optischer Durchbrüche, die die Schnittfläche bilden. Diese ununterbrochene Aneinanderreihung von Höhenlinien kann vorzugsweise dadurch erreicht werden, daß der Fokuspunkt jeweils bis auf ein Reststück vollständig entlang der Höhenlinie bewegt wird und im Reststück durch Verstellung des Fokuspunktes in der ersten Raumrichtung ein Übergang zur nächsten Höhenlinie bewirkt wird. Dieser Ansatz hat den Vorteil, daß die Anforderungen an die Verstellung in der ersten Raumrichtung nochmals gesenkt sind, da auch während des Übergangs zwischen zwei Höhenlinien optische Durchbrüche zur Bildung der Schnittfläche erzeugt werden. Das Höhenlinienbild wird dabei von der Topographie, d.h. der Krümmung der Schnittfläche abhängen. Bei einer sphärisch gekrümmten Schnittfläche erhält man konzentrische kreisförmige Höhenlinien. Da bei augenoptischen Korrekturen regelmäßig auch ein gewisser Astigmatismus zu korrigieren ist, wird jedoch eine sphärisch gekrümmte Schnittfläche eher die Ausnahme, eine Ellipsoid- oder Toroidfläche dagegen meist die Regel sein. Für eine solche Ellipsoidfläche sind die Höhenlinien erfindungsgemäß als (günstigerweise konzentrische) Ellipsen ausgebildet. Vorzugsweise liegt die Elliptizität zwischen 1,0 und 1,1 oder sogar 1,2.

Bei einer solchen Form werden die Höhenlinien auch derart der Führung des Fokuspunktes zugrundegelegt, daß der abgelenkte Fokuspunkt einer Ellipsoid-Spirale folgt, d.h. einer auf der Mantelfläche der gekrümmten Schnittfläche liegenden Spirale.

Die Elliptizität der Ellipsen bzw. der Ellipsoid-Spirale kann dabei von der Form der Hornhautoberfläche abhängen. Unter Elliptizität ist das Verhältnis der großen Hauptachse einer Ellipse zu ihrer kleinen Hauptachse zu verstehen.

Für kontaktlose Verfahren wird mit der natürlichen Oberflächentopographie gearbeitet, bei Verwendung eines Kontaktglases spielt die Form dieses Kontaktglases eine Rolle. Hier besteht ein Vorteil des Ansatzes mit Verwendung eines Kontaktglases, da die Topographie mit angepreßtem Kontaktglas wohl definiert ist. Ein ebenes Kontaktglas stellt einen mathematischen Grenzfall dar, und das Konzept des Höhenlinien-Scans führt hier zur Entartung der Bahnkurven, obwohl diese auch noch als geschlossen bezeichnet werden können. Für den auch aus applikativer Sicht interessanteren Fall eines gekrümmten Kontaktglases ergibt sich eine Abhängigkeit der Oberflächentopographie, z. B. der Elliptizität von der Krümmung des Kontaktglases. Dies gilt auch, wenn die Krümmung rein sphärisch ist, da sich dann ebenfalls eine Ellipsoidform für die Schnittfläche einstellt. Die Elliptizität ist allerdings meist nicht im ganzen Bearbeitungsfeld gleich, sondern zeigt oft eine radiale Abhängigkeit.

Im Prinzip ergibt sich für die Elliptizität e folgender Zusammenhang:

$$e(z) = \frac{\sqrt{R_a^2 - (R_a - z)^2}}{\sqrt{R_b^2 - (R_b - z)^2}},$$

wobei $R_a$ und $R_b$ die Krümmungsradien der Cornea-Oberfläche in Richtung der Hauptachsen der Ellipse bezeichnen und z der Abstand des Bearbeitungspunktes (der Höhenlinie) vom Hornhautscheitel ist. Da z dann eine Funktion des radialen Parameters des Bearbeitungsfeldes (Abstand zur optischen Achse) ist, ist es zweckmäßig, die bereits erwähnte radiale Abhängigkeit der Elliptizität als e(z) = e(z(r)) zu wählen.

Die oben genannte Gleichung gilt primär für das unkontaktierte Auge, da auch dort, wie oben erwähnt, meist eine Ellipsoidform vorliegt. Bei Anpressen an ein Kontaktglas kommt es in der Regel zu einer Deformation, die bei der Berechnung berücksichtigt wird. Dabei spielen neben den Kugelkoordinaten $R, \varphi, \alpha$ im natürlichen Augensystem und im Kontaktglassystem (gestrichene Koordinaten) der äußere Krümmungsradius der Cornea $R_{Cv}$ und der Krümmungsradius des Kontaktglases $R_G$ eine Rolle. Eine einfache und kompakte Form der Transformationsgleichungen für diese Anpreßtransformation lautet:

$$\varphi' = \varphi$$

$$\alpha' \cdot R' = \alpha \cdot R$$

$$R_G - R' = R_{Cv} - R$$

Weitere Ergänzungen, die in den Gleichungen zu Korrekturtermen führen, sind natürlich möglich und teilweise auch sinnvoll. Der hier offenbarte heuristische Ansatz wird dadurch jedoch nur modifiziert, gilt also im Prinzip weiter. Die genannten Zusammenhänge ermöglichen eine einfache Berechnung der Bahnkurven, was auch die Berechnung der Elliptizität mit einschließt. Ein besonders wichtiger Schritt bei den Algorithmen zur Berechnung ist die oben angegebene Vorwärts- und Rückwärtstransformation zwischen natürlichem Augensystem und Kontaktglassystem.

Die Elliptizität der Bahnkurven beträgt für ein Kontaktglas mit einem Krümmungsradius, der etwa dem des menschlichen Auges entspricht, in der Regel weniger als 1.4 (große Hauptachse 10% länger als die kleine Hauptachse). Die Elliptizität beträgt bei einer sphäro-zylindrischen Korrektur mit -2dpt und 1dpt beispielsweise nur etwa 1.03 im zentralen Feldbereich nahe der optischen Achse und wächst mit dem Abstand von der optischen Achse bis zur äußeren Bahnkurve um ca. 10%. Für eine praktikable Ausführungsform spielt die Veränderlichkeit der Elliptizität oder einer entsprechenden Modifikation einer idealen Kreisbahn bei Fehlsichtigkeitskorrektur höherer Ordnung keine störende Rolle, kann also in einer ersten Näherung als konstant angenommen werden.

Die Abstände zwischen den der Steuerung zugrunde zu legenden Höhenlinien sind naturgemäß durch die Abstände der Ebenen gegeben, die durch einen mathematischen Schnitt mit der gekrümmten Schnittfläche die Höhenlinien erzeugt sind. Um sicherzustellen, daß die Vielzahl von optischen Durchbrüchen eine zusammenhängende Schnittfläche ausbildet, ist darauf zu achten, daß der Maximalabstand der Höhenlinien einen Grenzwert nicht überschreitet. Es ist deshalb zweckmäßigerweise zu bevorzugen, daß Abstände der Höhenlinien in der ersten Raumrichtung so gewählt werden, daß die Abstände zwischen benachbarten Höhenlinien einen Grenzwert nicht überschreiten. Dabei kann als zu überprüfendes Maß sowohl der Abstand im Höhenlinienbild als auch der Abstand im dreidimensionalen Raum verwendet werden. Da in der Augenchirurgie die gekrümmten Schnittflächen zur optischen Korrektur innerhalb gewisser Grenzen in oftmals ausreichender Näherung einer sphärischen bzw. einer Ellipsoid-Geometrie genügen, kann es zur Vereinfachung ausreichen, daß die Abstände in der ersten Raumrichtung so gewählt werden, daß die mittleren Abstände der Höhenlinien konstant sind und insbesondere unterhalb eines Schwellwertes liegen, der natürlich geringer ist als der vorgenannte Grenzwert. Bei ellipsoidförmigen Schnittflächen kann vereinfacht im Höhenlinienbild der Abstand benachbarter Höhenlinien an der langen Halbachse ausgewertet werden, um sicherzustellen, daß die optischen Durchbrüche ausreichend dicht liegen.

Bei ophthalmologischen Operationen kann es mitunter erforderlich werden, auch höhere Aberrationen durch Entfernen eines Volumens aus der Hornhaut zu korrigieren. Die dazu erforderliche gekrümmte Schnittfläche weist dementsprechend dann auch höhere Krümmungsordnungen auf. Will man diese Formen durch Höhenlinien direkt abbilden, ergibt sich mitunter ein sehr komplexes Höhenlinienbild, das eine komplexe und schnelle Verstellung in den zwei übrigen Raumrichtungen beim Abfahren einer Höhenlinie erfordert. Für solche Fälle ist es zweckmäßig, bei der Bestimmung der Höhenlinien die höheren Krümmungsordnungen der gekrümmten Schnittfläche zu vernachlässigen und dann, während der Fokuspunkt in den übrigen zwei Raumrichtungen gemäß der Höhenlinie verstellt wird, die Verstellung in der ersten Raumrichtung gemäß dem Einfluß der höheren Krümmungsordnungen zu verändern. Die Korrektur höherer Aberrationen wird also dann in der ersten Raumrichtung, z.B. in z-Richtung auf eine Grundbewegung aufmoduliert, die der gekrümmten Schnittfläche ohne höhere Aberrationen entspricht.

Bei vielen augenoptischen Korrekturen ist es aufgrund physiologischer Gegebenheiten vorteilhaft, zur Fehlsichtigkeitskorrektur ein Volumen zu entnehmen, das bezogen auf die optische Achse des Auges in einem kreisförmigbegrenzten Bereich liegt. Dies gilt auch, falls astigmatische Korrekturen nötig sind. Für solche Fälle ist es vorteilhaft, mittels der Höhenlinien eine Ellipse abzutasten, in den Randbereichen, in denen die Ellipse über den gewünschten kreisförmigen Bereich hinausragt, jedoch die Laserstrahlung (z. B. durch einen optischen Schalter oder eine Blende oder durch Eingriff an der Laserstrahlungsquelle) so zu steuern, daß dort keine optischen Durchbrüche bewirkt werden. Durch eine derartige Ausblendung von Randbereichen der Ellipse kann sichergestellt werden, daß die (astigmatisch) gekrümmte Schnittfläche nur in einem kreisförmigen Bereich erzeugt wird.

In der erfindungsgemäßen Vorrichtung kann die Verstellung des Fokuspunktes mit einer Scaneinrichtung bewirkt werden, die zur Verstellung in der ersten Raumrichtung (üblicherweise z-Richtung) ein vorzugsweise als abstimmbares Teleskop ausgebildetes Zoom-Objektiv und für die anderen beiden Raumrichtungen (üblicherweise x- und y-Richtungen) zwei Kippspiegel mit gekreuzten Drehachsen aufweist.

Für die durch optische Mittel bewirkte Ausbildung gekrümmter Schnittflächen ist es vorteilhaft, wenn die Oberfläche des Materials, insbesondere die Augenhornautvorderfläche, eine bekannte Form hat. Dies erleichtert die Führung des Fokuspunktes. Weiter ist es zweckmäßig, das zu bearbeitende Material, insbesondere die Augenhornhaut, räumlich zu fixieren, da man dann auf mitunter aufwendige Strahlnachführungen verzichten kann. Unter beiden Gesichtspunkten ist es zweckmäßig, auf das Material ein Kontaktglas aufzusetzen, das der Materialoberfläche eine bestimmte Form gibt. Diese Form wird dann bei der Bestimmung der Höhenlinien berücksichtigt. Dies kann insbesondere dadurch erfolgen, daß die eingangs erwähnte Koordinatentransformation, die durch das Anpressen an das Kontaktglas erfolgt, bei der Ansteuerung eingeht.

Die Verwendung eines Kontaktglases ist sowohl für das beschriebene Verfahren als auch für die erfindungsgemäße Vorrichtung vorteilhaft. Bei der Vorrichtung ist die vom Kontaktglas der Oberfläche des Materials verliehene Form in der Steuereinrichtung bekannt oder wird dieser geeignet eingegeben, so daß die Steuereinrichtung bei der Wahl der Höhenlinien die Oberflächenform des Materials zugrundelegt.

Die Erfindung wird nachfolgend unter Bezugnahme auf die Zeichnung beispielhalber noch näher erläutert. In der Zeichnung zeigt:

| | |
|---|---|
| Figur 1 | eine perspektivische Darstellung eines Patienten während einer laserchirurgischen Behandlung mit einem laserchirurgischen Instrument, |
| Figur 2 | die Fokussierung eines Strahlenbündels auf das Auge des Patienten beim Instrument der Figur 1, |
| Figur 3 | eine schematische Darstellung zur Erläuterung einer während der laserchirurgischen Behandlung mit dem Instrument der Figur 1 erzeugten Schnittfläche, |
| Figur 4 | eine Ablenkvorrichtung des laserchirurgischen Instruments der Figur 1, |
| Figur 5 | ein beispielhaftes Höhenlinienbild, das bei der Ansteuerung der Ablenkeinrichtung der Figur 4 zugrundegelegt wird, |
| Figur 6 | einen Ausschnitt eines Höhenlinienbilds ähnlich dem der Figur 5 zur Verdeutlichung des Übergangs zwischen aufeinanderfolgenden Höhenlinien, |
| Figur 7 | ähnlich der Figur 6 mit einer weiteren Möglichkeit für einen Übergang zwischen Höhenlinien, |
| Figuren | 8a und 8b ein weiteres Beispiel für ein Höhenlinienbild samt zugehörigen Ansteuerfunktionen für die Ablenkvorrichtung der Figur 4, |
| Figur 9 | eine Draufsicht auf einen Schnittbereich beim Ausführen einer augenoptischen Operation zur Fehlsichtigkeitskorrektur, |
| Figur 10 | eine Darstellung ähnlich der Figur 2 bei Verwendung eines Kontaktglases, |
| Figur 11 | bei der Bestimmung der Höhenlinien relevante Größen und |
| Figuren 12 und 13 | die Größen der Figur 11 mit und ohne Kontaktglas. |

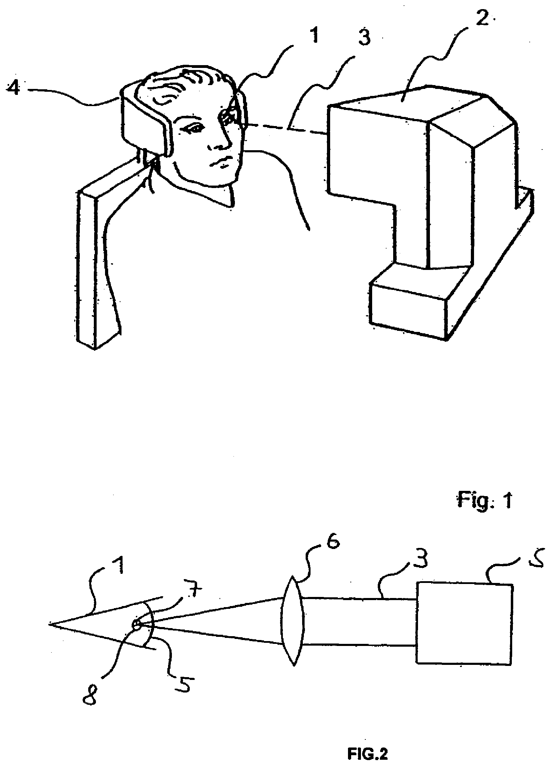

In Figur 1 ist ein laserchirurgisches Instrument zur Behandlung eines Auges 1 eines Patienten gezeigt, wobei das laserchirurgische Instrument 2 zur Ausführung einer refraktiven Korrektur dient. Das Instrument 2 gibt dazu einen Behandlungs-Laserstrahl 3 auf das Auge des Patienten 1 ab, dessen Kopf in einen Kopfhalter 4 fixiert ist. Das laserchirurgische Instrument 2 ist in der Lage, einen gepulsten Laserstrahl 3 zu erzeugen, so daß das in US 6.110.166 beschriebene Verfahren ausgeführt werden kann.

Das laserchirurgische Instrument 2 weist dazu, wie in Figur 2 schematisch dargestellt ist, eine Strahlquelle S auf, deren Strahlung in die Hornhaut 5 des Auges 1 fokussiert wird. Mittels des laserchirurgischen Instrumentes 2 wird eine Fehlsichtigkeit des Auges 1 des Patienten dadurch behoben, daß aus der Hornhaut 5 Material so entfernt wird, daß sich die Brechungseigenschaften der Hornhaut um ein gewünschtes Maß ändern. Das Material wird dabei dem Stroma der Hornhaut entnommen, das unterhalb von Epithel und Bowmanscher Membran oberhalb der Decemetschen Membran und des Endothels liegt.

Die Materialentfernung erfolgt, indem durch Fokussierung des hochenergetischen gepulsten Laserstrahls 3 mittels eines objektiven Teleskops 6 in einem in der Hornhaut 5 liegenden Fokus 7 in der Hornhaut Gewebeschichten getrennt werden. Jeder Puls der gepulsten Laserstrahlung 3 erzeugt dabei einen optischen Durchbruch im Gewebe, welcher eine Plasmablase 8 initiiert. Dadurch umfaßt die Gewebeschichttrennung ein größeres Gebiet, als der Fokus 7 der Laserstrahlung 3. Durch geeignete Ablenkung des Laserstrahls 3 werden nun während der Behandlung viele Plasmablasen 8 aneinandergereiht. Die aneinanderliegenden Plasmablasen 8 bilden dann eine Schnittfläche 9, die ein Teilvolumen T des Stromas, nämlich das zu entfernende Material der Hornhaut 5 umschreiben.

Das laserchirurgische Instrument 2 wirkt durch die Laserstrahlung 3 wie ein chirurgisches Messer, das, ohne die Oberfläche der Hornhaut 5 zu verletzen, direkt Materialschichten im Inneren der Hornhaut 5 trennt. Führt man den Schnitt durch weitere Erzeugung von Plasmablasen 8 bis an die Oberfläche der Hornhaut 5, kann ein durch die Schnittfläche 9 isoliertes Material der Hornhaut 5 seitlich herausgezogen und somit entfernt werden.

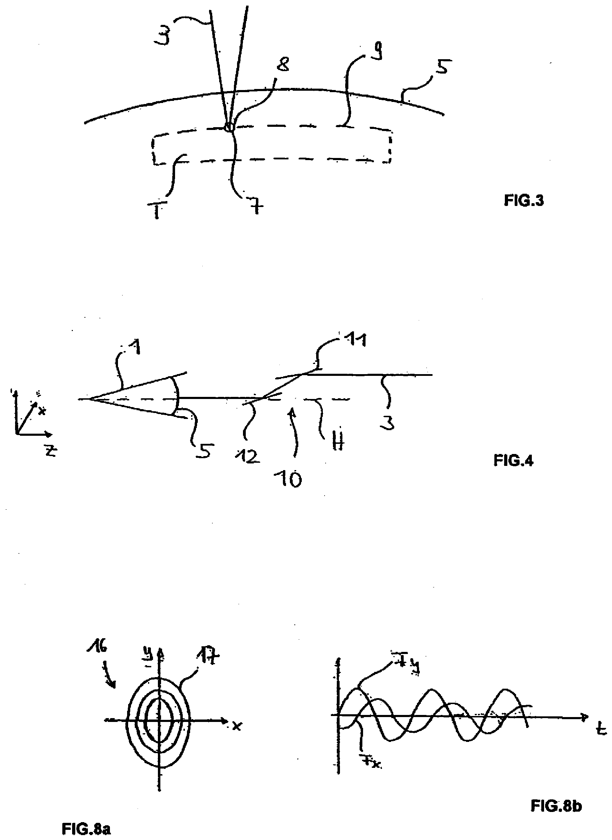

Die Erzeugung der Schnittfläche 9 mittels des laserchirurgischen Instrumentes 2 ist in Figur 3 schematisch dargestellt. Durch Aneinanderreihung der Plasmablasen 8 in Folge stetiger Verschiebung des Fokus 7 des gepulsten fokussierten Laserstrahls 3 wird die Schnittfläche 9 gebildet.

Die Fokusverschiebung erfolgt dabei zum einen in einer Ausführungsform mittels der in Figur 4 schematisch dargestellten Ablenkeinheit 10, die den auf einer Haupteinfallsachse H auf das Auge 1 einfallenden Laserstrahl 3 um zwei senkrecht zueinander liegenden Achsen ablenkt. Die Ablenkeinheit 10 verwendet dafür einen Zeilenspiegel 11 sowie einen Bildspiegel 12, was zu zwei hintereinander liegenden räumlichen Ablenkachsen führt. Der Kreuzungspunkt der Hauptstrahlachse mit der Ablenkachse ist dann der jeweilige Ablenkpunkt. Zur Fokusverschiebung wird zum anderen das Teleskop 6 geeignet verstellt. Dadurch kann der Fokus 7 in dem in Figur 4 schematisch dargestelltem x/y/z-Koordinatensystem entlang dreier orthogonaler Achsen verstellt werden. Die Ablenkeinheit 10 verstellt den Fokus in der x/y-Ebene, wobei der Zeilenspiegel den Fokus in der x-Richtung und der Bildspiegel in der y-Richtung zu verstellen erlaubt. Das Teleskop 6 wirkt dagegen auf die z-Koordinate des Fokus 7.

Ist eine wie in Fig. 3 gezeigte Schnittfläche 9 in die gleiche Richtung wie die Hornhautoberfläche gewölbt, so ist dies mit einer Optik, deren Bildfeldkrümmung ähnlich der Krümmung der Hornhaut ist, zu erreichen, ohne daß die Führung des Fokus 7 dies berücksichtigen muß.

Aufgrund der Corneakrümmung, die zwischen 7 und 10 mm beträgt, ist das Teilvolumen T auch entsprechend gekrümmt. Die Corneakrümmung wirkt sich somit in Form einer Bildfeldkrümmung aus. Diese wird durch geeignete Ansteuerung der Ablenkeinheit berücksichtigt.

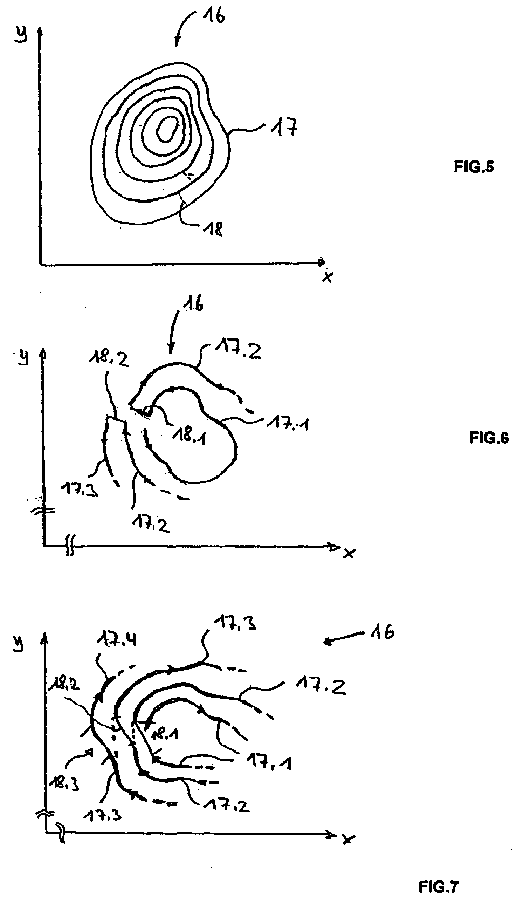

Zur Erzeugung der Schnittfläche 9 wird aus deren Krümmung ein Höhenlinienbild 16 bestimmt, wie es beispielshalber in Figur 5 in der x/y-Ebene dargestellt ist. Das Höhenlinienbild 16 besteht aus einer Vielzahl konzentrischer Höhenlinien 17, die Punkte gleicher z-Koordinaten der Schnittfläche 9 verbindet. Das Höhenlinienbild 16 wurde gewonnen, in dem aus der gekrümmten Schnittfläche 9 diejenigen Punkte bestimmt, z. B. herausgefiltert wurden, die zumindest näherungsweise eine bestimmte z-Koordinate haben. Dies entspricht einem mathematischen Schnitt der gekrümmten Schnittfläche 9 mit einer x/y-Ebene mit der jeweiligen z-Koordinate. Die z-Koordinaten wurden dabei zur Erzeugung der einzelnen Höhenlinien 17 des Höhenlinienbildes 16 der Figur 5 so gewählt, daß die Abstände benachbarter der Höhenlinien 17 im Höhenlinienbild 16 einen vorbestimmten Grenzwert nicht überschreiten. Dieser Grenzwert ist durch den maximal zulässigen Abstand zweier Plasmablasen 8 festgelegt, der zum Erreichen einer zusammenhängenden Schnittfläche zulässig ist.

Zum Erzeugen der Schnittfläche 9 wird nun der Fokus 7 entsprechend der Höhenlinien 17 durch die Ablenkeinheit 10 verstellt, wobei die Zoom-Optik 6 für jede Höhenlinie 17 die entsprechende z-Koordinate für den Fokus 7 einstellt. Während der Fokus 7 über eine Höhenlinie 17 läuft, bleibt das Teleskop 6 fest eingestellt. Lediglich während in Figur 5 gestrichelt eingezeichneten Übergängen 18 zwischen benachbarten Höhenlinien erfolgt eine Verstellung.

Figur 6 zeigt einen Ausschnitt des Höhenlinienbildes 16. Jede Höhenlinie 17 wird dabei als fast vollständig geschlossene Kurve vom Fokus 7 abgefahren, wobei der Abstand zwischen Anfang und Ende einer Höhenlinie 17 den durch den Grenzwert definierten zulässigen maximalen Abstand zwischen zwei Plasmablasen 8 nicht überschreitet. Am Ende einer jeden Höhenlinie 17 (in Figur 6 sind drei Höhenlinien 17.1, 17.2 und 17.3 angedeutet) erfolgt ein Übergang 18 durch Verstellen des Teleskopes 6 zur jeweils nächsten Höhenlinie. Zwischen den Höhenlinien 17.1 und 17.2 liegt dadurch ein Übergang 18.1, zwischen den Höhenlinien 17.2 und 17.3 ein Übergang 18.2. Dies setzt sich für alle Höhenlinien fort. Durch den derart gewählten Übergang ist zum einen erreicht, daß der Grenzwert für den maximal zulässigen Abstand zwischen zwei Plasmablasen 8 nicht überschritten wird, zum anderen die Höhenlinien 17 als zusammenhängende Spur geschrieben werden kann.

In Figur 6 liegen die Übergänge 18 im wesentlichen auf Fallinien der gekrümmten Schnittfläche 9. Figur 7 zeigt diesbezüglich andere Übergänge 18.1 bis 18.3, bei denen ein gleitender Übergang zwischen dem Ende einer Höhenlinie und dem Beginn der unmittelbar benachbarten Höhenlinie erfolgt. Zur Verdeutlichung ist die vom Fokus 7 nicht verfolgte Fortsetzung der entsprechenden Höhenlinien in Figur 7 gestrichelt eingezeichnet. Wie zu sehen ist, wird am Ende einer Höhenlinie 17 ein gleitender Übergang auf die nächste Höhenlinie durch geeignete Ansteuerung des Zeilenspiegels 11 sowie des Bildspiegels 12 vorgenommen. Gleichzeitig wird synchron das Teleskop 6 während der dadurch erreichten Übergänge 18.1, 18.2 und 18.3 verstellt.

Es ergibt sich dadurch im Gegensatz zum Übergang der Figur 6, bei der benachbarte Höhenlinien in entgegengesetzter Umlaufrichtung durchlaufen werden, ein gleichsinniger Umlauf um die Höhenlinien, die ähnlich einer Spirale aneinandergereiht werden. Im Unterschied zu einer wirklichen Spirale wird jedoch bis auf den Übergang 18 die Höhenlinie durch den Fokus 7 abgefahren und der Wechsel von einer Höhenlinie zur nächsten erfolgt über einen kleinen Winkelbereich des Umlaufs statt kontinuierlich während eines 360° Umlaufes.

Figur 8a zeigt ein weiteres Beispiel für ein Höhenlinienbild 16, das hier aus konzentrischen elliptischen Höhenlinien 17 aufgebaut ist. Für dieses Höhenlinienbild ist für jede Höhenlinie 17 die in Figur 8b schematisch dargestellte zeitliche Ansteuerung von Zeilenspiegel 11 und Bildspiegel 12 vorgesehen, die hier mit Ansteuerfunktionen Fy und Fx angesteuert werden, die der Gleichung $\sin\varphi$ bzw. $A \cdot \sin(\varphi+\alpha)$ und $\cos\varphi$ bzw. $R \cdot \cos(\varphi+\alpha)$ genügen (mit $\varphi$ Winkelparameter der Höhenlinie, $\alpha$ auf die Winkellage der Ellipsenhauptachse zur y-Achse wirkendem Parameter R, und A die Elliptizität beeinflussendem Parameter, wobei meist R=1 gilt).

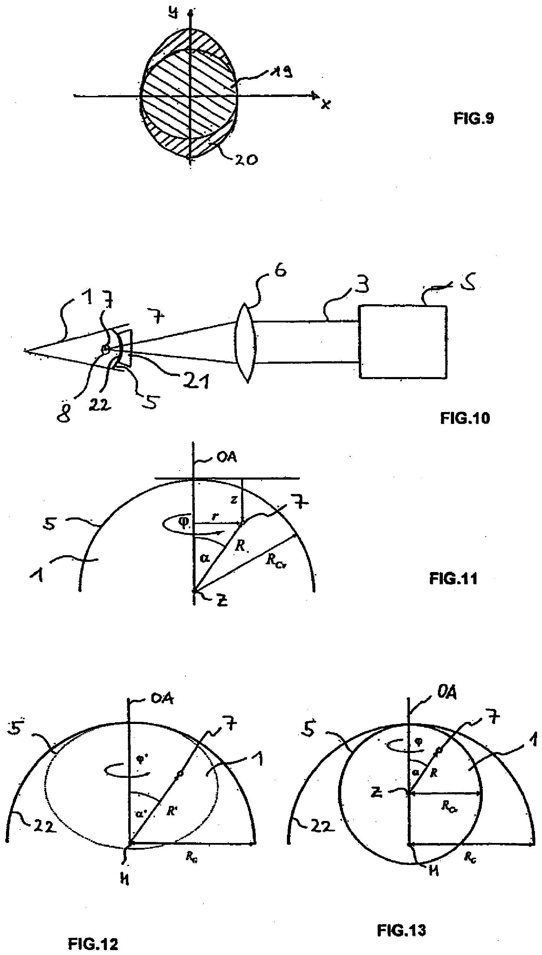

Da bei einem nichtkreisförmigen Höhenlinienbild die Schnittfläche 9, in z-Richtung gesehen einen nichtkreisförmigen Bereich umfaßte, was aus ophtalmologischer Sicht nicht wünschenswert ist, wird in einer Ausführungsform bei solchen, nicht rotationssymmetrischen Höhenlinienbildern in Bereichen, die außerhalb eines kreisförmigen Bereiches liegen, die Strahlquelle S so gesteuert, daß sie im Material 5 keinen optischen Durchbruch, d.h. keine Plasmablase 8 erzeugt. Dies ist in Figur 9 durch unterschiedliche Schraffuren dargestellt. Im von links oben nach rechts unten schraffierten kreisförmigen Bereich 19 kann die Strahlquelle S Plasmablasen 8 erzeugen. In den darüber hinausragenden Bereichen 20, in denen das Höhenlinienbild 16 den gewünschten kreisförmigen Bereich 19 verläßt, ist die Strahlquelle S dagegen abgeschaltet oder wird zumindest so betrieben, daß keine Plasmablasen 8 entstehen können.

Bisher wurde das laserchirurgische Instrument 2 sowie das dadurch ausgeführte Verfahren in Zusammenhang mit einem Konzept beschrieben, das die Form der Vorderfläche der Augenhornhaut für den Eingriff unverändert läßt. Obige Beschreibung gilt jedoch auch für Ansätze, die auf die Augenhornhaut 5 ein Kontaktglas aufsetzen. Der bei einem solchen Vorgehen vorliegende Aufbau ist schematisch in Figur 10 gezeigt, die im wesentlichen der Figur 2 entspricht, so daß auf dort bereits beschriebene Elemente nicht weiter eingegangen wird. Im Unterschied zu Figur 2 ist auf die Augenhornhaut 5 jetzt allerdings ein Kontaktglas 21 aufgesetzt, das mit seiner Innenfläche 22 der Vorderfläche der Augenhornhaut 5 ein bestimmtes Profil verleiht. Im Unterschied zum bisher beschriebenen Vorgehen, ist nun bei der Bestimmung der Bahnkurven, z. B. der Höhenlinien, nicht mehr die Krümmung der Augenhornhaut 5 im freien, d. h. natürlichen Zustand, zugrundezulegen, sondern die durch die Innenfläche 22 des Kontaktglases 21 vorgegebene Form.

Ohne Kontaktglas 21 stellen sich die geometrischen Verhältnisse am Auge 1 wie in Figur 11 gezeigt dar. Die Augenhornhaut 5 ist bezogen auf das Augenzentrum Z näherungsweise sphärisch gekrümmt, so daß sie durch einen Krümmungsradius $R_{CV}$ sowie die Lage des Zentrums Z auf der optischen Achse OA eindeutig lagebestimmt ist. Die Koordinaten eines Punktes, an dem ein Laserfokus 7 zu liegen kommt, um eine Plasmablase 8 zu erzeugen, sind damit entweder in Zylinderkoordinaten (Radius r von der optischen Achse OA, Abstand z von der Scheitelpunktebene und Winkel $\varphi$) oder in radialen Koordinaten (Radius r vom Augenzentrum Z, Winkel $\varphi$ und $\alpha$) eindeutig angebbar. In beiden Koordinatensystemen können die Höhenlinien bzw. die Bahnkurven, an denen der Fokus 7 verstellt wird, berechnet und angegeben werden, wobei in Zylinderkoordinaten elliptische Bahnkurven besonders einfach mathematisch beschrieben werden können.

Setzt man nun auf das Auge ein Kontaktglas 21 auf, liegen die in Figur 13 gezeigten Verhältnisse vor, solange das Kontaktglas 21 mit seiner Innenfläche 22 die Augenhornhaut nicht verformt. Das Kontaktglas ist hier sphärisch gekrümmt, wobei der Krümmungsradius $R_G$ größer ist, als der Krümmungsradius $R_{CV}$ der Augenhornhaut. Preßt man nun das Kontaktglas 21 auf das Auge 1, verformt sich die Augenhornhaut 5 von einer Sphäre zu einem Ellipsoid; es stellen sich die in Figur 12 schematisch dargestellten Verhältnisse ein. Das Anpressen bewirkt also die Verformung des Auges, das sich zumindest in einem Bereich um die optische Achse OA sehr viel enger an die Innenfläche 22 des Kontaktglases 21 anlegt, als ohne Anpressung.

Da sich die geometrischen Verhältnisse nun ändern, kann man den Vorgang des Anpressens bezüglich der mathematischen Beschreibung der Orte der Fokuspunkte 7 und damit der Bahnkurven in einer Koordinatentransformation, die auch als "Anpreßtransformation" bezeichnet wird, fassen. Die transformierten Koordinaten werden dann zweckmäßigerweise auf den Mittelpunkt M des vorzugsweise sphärisch gekrümmten Kontaktglases bezogen, da das Kontaktglas üblicherweise auch zur Fixierung des Auges 1 verwendet wird, d. h. fest mit dem Instrument 2 verbunden ist. Hier wirkt sich die Doppelfunktion des Kontaktglases (Formgebung und räumliche Fixierung) aus.

Es stellen sich dann elliptische Bahnkurven ein. Die Elliptizität der Bahnkurven hängt von der Form diese Kontaktglases ab. Unter Elliptizität ist das Verhältnis der großen Hauptachse einer Ellipse zu ihrer kleinen Hauptachse zu verstehen.

Ein ebenes Kontaktglas stellt einen mathematischen Grenzfall dar, und das Konzept des Höhenlinien-Scans führt hier zur Entartung der Bahnkurven, obwohl diese auch noch als geschlossen bezeichnet werden können. Für den auch aus applikativer Sicht interessanteren Fall eines gekrümmten Kontaktglases ergibt sich eine Abhängigkeit der Elliptizität von der Krümmung des Kontaktglases. Die Elliptizität ist zudem meist nicht im ganzen Bearbeitungsfeld gleich, sondern zeigt eine radiale Abhängigkeit.

Im Prinzip gilt für die Elliptizität e:

$$e(z) = \frac{\sqrt{R_a^2 - (R_a - z)^2}}{\sqrt{R_b^2 - (R_b - z)^2}},$$

wobei $R_a$ und $R_b$ die Krümmungsradien der Cornea-Oberfläche in Richtung der Hauptachsen der Ellipse bezeichnen und z den Abstand des Bearbeitungspunktes (der Höhenlinie) vom Hornhautscheitel. Da z in dem gewählten Zylinderkoordinatensystem (z, Abstand von Hornhautscheitel; r, Abstand zur optischen Achse; $\varphi$) dann eine Funktion des radialen Parameters v des Bearbeitungsfeldes ist, ist es zweckmäßig, die radiale Abhängigkeit der Elliptizität durch $e(z) = e(z(r))$ zu beschreiben.

Die oben genannte Gleichung gilt primär für das unkontaktierte Auge. Bei Anpressen an ein Kontaktglas kommt es in der Regel zu einer Deformation, die bei der Berechnung berücksichtigt werden muß. Dabei spielen der äußere Krümmungsradius der Cornea $R_{Cv}$ und der Krümmungsradius des Kontaktglases $R_G$ eine Rolle. Eine einfache und kompakte Form der Transformation lautet:

$$\varphi' = \varphi$$

$$\alpha' \cdot R' = \alpha \cdot R$$

$$R_G - R' = R_{Cv} - R$$

Weitere Ergänzungen, die in den Gleichungen zu Korrekturtermen führen, sind natürlich möglich und teilweise auch sinnvoll. Obiger Ansatz wird dadurch jedoch nur modifiziert, gilt also im Prinzip weiter. Die genannten Zusammenhänge ermöglichen die Berechnung der Bahnkurven, was auch die Berechnung der Elliptizität mit einschließt. Ein besonders wichtiger Schritt bei den Algorithmen zur Berechnung ist die Vorwärts- und Rückwärtstransformation zwischen natürlichem Augensystem und Kontaktglassystem.

Die Elliptizität der Bahnkurven beträgt für ein Kontaktglas mit einem Krümmungsradius, der etwa dem des menschlichen Auges entspricht, in der Regel weniger als 1.2 (große Hauptachse 10% länger als die kleine Hauptachse). Die Elliptizität beträgt bei einer sphäro-zylindrischen Korrektur mit -2dpt und 1dpt beispielsweise nur etwa 1.03 im zentralen Feldbereich nahe der optischen Achse und wächst mit dem Abstand von der optischen Achse bis zur äußeren Bahnkurve um ca. 10%. Für einen Ansatz spielt die Veränderlichkeit der Elliptizität oder einer entsprechenden Modifikation einer idealen Kreisbahn bei Fehlsichtigkeitskorrektur höherer Ordnung keine störende Rolle, kann also in einer ersten Näherung als konstant angenommen werden.

Es sei noch einmal betont, daß die erfindungsgemäße Verwendung von Höhenlinien sowohl für Ansätze mit wie auch ohne (ebenem oder gekrümmtem) Kontaktglas anwendbar sind, allerdings entfällt bei der Verwendung eines Kontaktglases jeglicher Nachführungsbedarf und es besteht keine Unsicherheit hinsichtlich der vorliegenden Oberflächentopographie.

Wird kein Kontaktglas verwendet, läßt sich die Oberflächentopographie mit geeigneten Verfahren und Vorrichtungen bestimmen und wird anschließend, (analog) ebenso wie die durch den Anpreßvorgang festgelegte Topographie im Verfahren berücksichtigt.

Ist die Form der Kontaktglasfläche nicht durch eine Sphäre beschreibbar, sondern folgt einer anderen Raumflächenfunktion, beispielsweise einem Paraboloiden, kann analog der oben angegebenen Transformation ein Transformationsgesetz angegeben werden, das aber den gleichen physikalischen Gedanken folgt.

## Patentansprüche

1. Vorrichtung zum Ausbilden einer ellipsoidischen Schnittfläche (9) in der Augenhornhaut (5), mit einer Laserstrahlungsquelle (S), die Laserstrahlung (3) in die Augenhornhaut (5) fokussiert und dort optische Durchbrüche (8) bewirkt, wobei eine Scaneinrichtung (6, 10), die den Fokuspunkt (7) dreidimensional verstellt, und eine Steuereinrichtung (2) vorgesehen sind, die die Scaneinrichtung (6, 10) ansteuert, um die Schnittfläche (9) durch Aneinanderreihen der optischen Durchbrüche (8) in der Augenhornhaut (5) zu bilden, und wobei die Scaneinrichtung (6, 10) zur Verstellung des Fokuspunktes (7) in einer ersten Raumrichtung (z) eine verstellbare Optik (6) aufweist, **dadurch gekennzeichnet, dass** die Steuereinrichtung (2) die Scaneinrichtung (6, 10) so ansteuert, daß die Steuereinrichtung der Führung des Fokuspunktes (7) elliptische Höhenlinien mit einer Elliptizität größer 1,0 zugrundelegt, so dass der Fokuspunkt (7) längs einer in der ellipsoidischen Schnittfläche liegenden Ellipsoid-Spirale geführt ist.

2. Vorrichtung nach Anspruch 1, **dadurch gekennzeichnet, dass** die Höhenlinien elliptisch mit einer Elliptizität kleiner gleich 1,2 sind.

3. Vorrichtung nach einem der obigen Vorrichtungsansprüche, **dadurch gekennzeichnet, dass** mittels eines Kontaktglases (21) der Oberfläche der Augenhornhaut (5) eine bestimmte Form verliehen ist und dass die Steuereinrichtung (2) die bestimmte Form bei der Ellipsoid-Spirale berücksichtigt.

4. Vorrichtung nach einem der obigen Vorrichtungsansprüche, **dadurch gekennzeichnet, dass** das Kontaktglas (21) gekrümmt ist und dass die Steuereinrichtung (2) die bestimmte Form bei der Ellipsoid-Spirale berücksichtigt, indem eine durch das Kontaktglas (21) bewirkte Verformung der Augenhornhaut (5) durch eine Koordinatentransformation berücksichtigt wird, die lautet

$$\varphi' = \varphi$$

$$\alpha' \cdot R' = \alpha \cdot R$$

$$R_G - R' = R_{Cv} - R$$

wobei $\alpha$, $\varphi$, R Kugelkoordinaten vor der Verformung der Augenhornhaut (5), $\alpha'$, $\varphi'$, R' Kugelkoordinaten nach der Verformung der Augenhornhaut (5), $R_{Cv}$ ein äußerer Krümmungsradius der unverformten Augenhornhaut (5) und $R_G$ ein Krümmungsradius des Kontaktglases (21) sind.

5. Vorrichtung nach einem der obigen Vorrichtungsansprüche, **dadurch gekennzeichnet, dass** die Steuereinrichtung (2) die Laserstrahlung (3) bezüglich der Erzeugung optischer Durchbrüche (8) abschaltet, solange die Höhenlinie (17) außerhalb eines Sollbereiches des Materials (5) verläuft, in dem die Schnittfläche (9) erzeugt werden soll, wobei insbesondere der Sollbereich (19) entlang der ersten Raumrichtung (z) gesehen kreisförmig ist.

6. Vorrichtung nach einem der obigen Vorrichtungsansprüche, **gekennzeichnet durch** eine Einrichtung zum kurzzeitigen Abschalten oder Schwächen des Laserstrahls (3).

7. Vorrichtung nach Anspruch 1, **dadurch gekennzeichnet, dass** die verstellbare Optik eine Teleskopanordnung (6) aufweist.

8. Vorrichtung nach einem der obigen Vorrichtungsansprüche, **dadurch gekennzeichnet, dass** die Scaneinrichtung (6, 10) zwei Kippspiegel (11, 12) mit gekreuzten Drehachsen aufweist, um die Verstellung in den übrigen zwei Raumrichtungen (x, y) zu bewirken.

9. Vorrichtung nach einem der obigen Vorrichtungsansprüche, dadurch gegenzeichnet, dass die Steuereinrichtung (2) bei höheren Krümmungsordnungen der Schnittfläche (9) die Höhenlinien (17) durch Schneiden einer um höhere Krümmungsordnungen bereinigten gekrümmten Schnittflächen (9) mit Ebenen senkrecht zur ersten Raumrichtung (z) bestimmt.

10. Vorrichtung nach Anspruch 9, dadurch gegenzeichnet, dass die Steuereinrichtung (2) die Verstellung in der ersten Raumrichtung (z) gemäß dem Einfluß der höheren Krümmungsordnungen verändert, während sie den Fokuspunkt (7) in den übrigen zwei Raumrichtungen (x, y) gemäß der Höhenlinien (17) verstellt, die der bereinigten Schnittfläche (9) ohne höhere Krümmungsordnungen zugeordnet sind.

**Claims**

1. An apparatus for forming an ellipsoidal cut surface (9) in the cornea of the eye (5), the apparatus comprising a laser radiation source (S) which focuses laser radiation (3) into the cornea of the eye (5) and generates optical breakthroughs (8) in the cornea, wherein a scanning unit (6, 10) which three-dimensionally shifts the focal point (7) and a control unit (2) which controls the scanning unit (6, 10) are provided, in order to form the cut surface (9) by sequential arrangement of the optical breakthroughs (8) in the cornea of the eye (5), and wherein the scanning unit (6, 10) comprises adjustable optics (6) for shifting the focal point (7) in one first spatial direction (z), **characterized in that** the control unit (2) controls the scanning unit (6, 10) such that the control unit bases the guidance of the focal point (7) on elliptical contour lines having an ellipticity greater than 1.0 such that the focal point (7) is guided along an ellipsoidal spiral located in the ellipsoidal cut surface.

2. The apparatus according to claim 1, **characterized in that** the contour lines are elliptical with an ellipticity of less than or equal to 1.2.

3. The apparatus according to any of the above apparatus claims, **characterized in that** by way of a contact glass (21) a particular shape (22) is imparted to the surface of the cornea of the eye (5), and that the control unit (2) considers said particular shape for the ellipsoidal spiral.

4. The apparatus according to any of the above apparatus claims, **characterized in that** the contact glass (21) is curved and that the control unit (2) considers the particular shape for the ellipsoidal spiral by way of considering a deformation of the cornea of the eye (5) created by the contact glass (21) through a coordinate transformation, which reads

$$\varphi' = \varphi$$

$$\alpha' \cdot R' = \alpha \cdot R$$

$$R_G - R' = R_{Cv} - R$$

wherein $\alpha$, $\varphi$, R are spherical coordinates prior to the deformation of the cornea of the eye (5), $\alpha'$, $\varphi'$, R' are spherical coordinates after the deformation of the cornea of the eye (5), $R_{Cv}$ is an anterior radius of curvature of the undeformed cornea of the eye (5) and $R_G$ is a radius of curvature of the contact glass (21).

5. The apparatus according to any of the above apparatus claims, **characterized in that** the control unit (2) deactivates the laser radiation (3) with respect to generating optical breakthroughs (8), as long as the contour line (17) extends outside a desired region of the material (5) in which the cut surface (9) is to be produced, said desired region (19) being, in particular, circular as viewed along the first spatial direction (z).

6. The apparatus according to any of the above apparatus claims, **characterized by** a unit for temporary deactivation or attenuation of the laser beam (3).

7. The apparatus according to claim 1, **characterized in that** the adjustable optics comprise a telescope arrangement (6).

8. The apparatus according to any of the above apparatus claims, **characterized in that** the scanning unit (6, 10) comprises two tilting mirrors (11, 12) with crossed axes of rotation in order to effect the shift in the two other spatial directions (x, y).

9. The apparatus according to any of the above apparatus claims, **characterized in that**, in case of higher orders of curvature of the cut surface (9), the control unit (2) determines the contour lines (17) by sectioning a curved cut surface (9), which is corrected with regard to higher orders of curvature, with planes perpendicular to the first spatial direction (z).

10. The apparatus according to claim 9, **characterized in that** the control unit (2) modifies the shift in the first spatial direction (z) according to the influence of the higher orders of curvature, while said control unit shifts the focal point (7) in the two other spatial directions (x, y) according to the contour lines (17) which are assigned to the cut surface (9) corrected without higher orders of curvature.

**Revendications**

1. Dispositif pour former une surface de coupe ellipsoïdale (9) dans la cornée de l'oeil (5), avec une source de rayonnement laser (S), qui focalise un rayonnement laser (3) dans la cornée de l'oeil (5) et y crée des passages optiques (8), dans lequel il est prévu un dispositif de balayage (6, 10), qui déplace dans les trois dimensions le point focal (7), et un dispositif de commande (2), qui commande le dispositif de balayage (6, 10), afin de former la surface de coupe (9) en enchaînant les passages optiques (8) dans la cornée de l'oeil (5), et dans lequel le dispositif de balayage (6, 10) présente une optique déplaçable (6) pour le déplacement du point focal (7) dans une première direction de l'espace (z), **caractérisé en ce que** le dispositif de commande (2) commande le dispositif de balayage (6, 10) de telle manière que le dispositif de commande base le guidage du point focal (7) sur des lignes de niveau elliptiques avec une ellipticité supérieure à 1,0, de telle manière que le point focal (7) soit conduit le long d'une spirale ellipsoïdale située dans la surface de coupe ellipsoïdale.

2. Dispositif selon la revendication 1, **caractérisé en ce que** les lignes de niveau sont elliptiques avec une ellipticité inférieure ou égale à 1,2.

3. Dispositif selon l'une quelconque des revendications de dispositif précédentes, **caractérisé en ce qu'**une forme déterminée est conférée à la surface de la cornée de l'oeil (5) au moyen d'un verre de contact (21) et **en ce que** le dispositif de commande (2) tient compte de la forme déterminée pour la spirale ellipsoïdale.

4. Dispositif selon l'une quelconque des revendications de dispositif précédentes, **caractérisé en ce que** le verre de contact (21) est courbe et **en ce que** le dispositif de commande (2) tient compte de la forme déterminée pour la spirale ellipsoïdale, par le fait qu'une déformation de la cornée de l'oeil (5) provoquée par le verre de contact (21) est prise en compte par une transformation de coordonnées, qui s'écrit

$$\varphi' = \varphi$$

$$\alpha' \cdot R' = \alpha \cdot R$$

$$R_G - R' = R_{Cv} - R$$

où $\alpha$, $\varphi$, $R$ sont les coordonnées sphériques avant la déformation de la cornée de l'oeil (5), $\alpha'$, $\varphi'$, $R'$ sont les coordonnées sphériques après la déformation de la cornée de l'oeil (5), $R_{Cv}$ est un rayon de courbure extérieur de la cornée de l'oeil (5) non déformée et $R_G$ est un rayon de courbure du verre de contact (21).

5. Dispositif selon l'une quelconque des revendications de dispositif précédentes, **caractérisé en ce que** le dispositif de commande (2) coupe le rayonnement laser (3) en ce qui concerne la production de passages optiques (8), aussi longtemps que la ligne de niveau (17) se situe à l'extérieur d'une région théorique de la matière (5), dans laquelle la surface de coupe (9) doit être produite, dans lequel en particulier la région théorique est de forme circulaire, considérée le long de la première direction de l'espace (z).

6. Dispositif selon l'une quelconque des revendications de dispositif précédentes, **caractérisé par** un système pour la coupure ou l'atténuation instantanée du faisceau laser (3).

7. Dispositif selon la revendication 1, **caractérisé en ce que** l'optique déplaçable présente un agencement télescopique (6).

8. Dispositif selon l'une quelconque des revendications de dispositif précédentes, **caractérisé en ce que** le dispositif de balayage (6, 10) présente deux miroirs basculants (11, 12) avec des axes de rotation croisés, afin de provoquer le déplacement dans les deux autres directions de l'espace (x, y).

9. Dispositif selon l'une quelconque des revendications de dispositif précédentes, **caractérisé en ce que** le dispositif de commande (2) détermine, pour des degrés de courbure plus élevés de la surface de coupe (9), les lignes de niveau (17) en coupant une surface de coupe courbe (9) corrigée de degrés de courbure plus élevés avec des plans perpendiculaires à la première direction de l'espace (z).

10. Dispositif selon la revendication 9, **caractérisé en ce que** le dispositif de commande (2) change le déplacement dans la première direction de l'espace (z) selon l'influence des degrés de courbure plus élevés, tandis qu'il déplace le point focal (7) dans les deux autres directions de l'espace (x, y) selon les lignes de niveau (17), qui sont associées à la surface de coupe corrigée (9) sans degrés de courbure plus élevés.

Fig. 1

FIG.2

FIG.3

FIG.4

FIG.8a

FIG.8b

FIG.5

FIG.6

FIG.7

FIG.9

FIG.10

FIG.11

FIG.12

FIG.13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5984916 A
- US 6110166 A
- US 5993438 A
- WO 9409849 A
- US 6325792 B